Europäisches Patentamt

**European Patent Office**

**Office européen des brevets**

(19)

(11) Publication number: **0 071 650**
**A1**

(12)

# EUROPEAN PATENT APPLICATION

published in accordance with Art. 158(3) EPC

(21) Application number: **82900564.4**

(22) Date of filing: **12.02.82**

Data of the international application taken as a basis:

(86) International application number: **PCT/JP 82/00041**

(87) International publication number: **WO 82/02951 (02.09.82 82/21)**

(51) Int. Cl.³: **G 01 N 21/75**, G 01 N 33/72, C 12 Q 1/28

(30) Priority: **16.02.81 JP 21851/81**

(43) Date of publication of application: **16.02.83 Bulletin 83/7**

(84) Designated Contracting States: **AT BE CH DE FR GB LI NL SE**

(71) Applicant: **Fujisawa Pharmaceutical Co., Ltd., 3, Doshomachi 4-chome Higashi-ku, Osaka-shi, Osaka 541 (JP)**

(72) Inventor: **OGOSHI, Yoshimasa, 15, Koaza-Furuyashiki Oaza-Hosono, Seika-cho Soraku-gun Kyoto 619-02 (JP)**

(74) Representative: **Bühling, Gerhard, Dipl.-Chem. et al, Patentanwaltsbüro Tiedtke-Bühling-Kinne Grupe-Pellmann Bavariaring 4, D-8000 München 2 (DE)**

(54) **APPARATUS FOR MEASURING CONCENTRATION OF BILIRUBIN.**

(57) An apparatus having optical means for measuring the concentration of total bilirubin (TB) contained in a serum and the concentration of bilirubin not coupled to albumin (UB). The intensity of monochromatic light of a predetermined wavelength incident and transmitted from a light source (2) to a specimen (serum) in a cell (10) contained in a constant temperature oven unit (1) is converted through a photodetector (3) and a bilirubin measuring unit (4) into the TB concentration, stored in a TB concentration memory circuit (5) and indicated on a indicator (7). A UB concentration measuring unit (6) serves to measure the speed of variation of the output of the bilirubin measuring unit (4) obtained by adding peroxidase and a substance having a function of producing hydrogen peroxide to the serum in the cell in order to calculate the UB concentration and to indicate it on an indicator (8). The respective members are associated integrally within an outer housing (9), and the TB and UB concentrations in the serum can be simultaneously measured.

# APPARATUS FOR MEASURING CONCENTRATIONS OF BILIRUBIN

## TECHNICAL FIELD

The present invention relates to a bilirubin concentration measuring apparatus for use in clinical tests for optically measuring the concentration of total bilirubin (hereinafter abbreviated as "TB") contained in the serum and the concentration of bilirubin not bound to albumin (unbound bilirubin, hereinafter referred to briefly as "UB").

## BACKGROUND ART

The problem of infants with cerebral nerve disturbances has raised a great social issue in recent years. To predict the hazard of kernicterus in newborn infants, a cause of such disturbances, it is important to detect the concentration of UB which is toxic to the central nervous system. It has been clinically clarified that kernicterus can be diagnosed more accurately by measuring the UB concentration simultaneously with the measurement of the TB concentration which has been conducted conventionally. It is known that in the presence of hydrogen peroxide, peroxidase acts to readily oxidize serum UB but has difficulty in oxidizing bilirubin bound to albumin and that the rate of reduction in the amount of serum bilirubin is in a definite proportional relation to the UB concentration. It is also known to measure the UB concentration of the serum by causing peroxidase to act on serum bilirubin in the presence of hydrogen peroxide, determining the rate of reduction in the amount of serum bilirubin from the resulting variation in the absorbance for monochromatic light of a predetermined wavelength and calculating the UB concentration from the reduction rate. However, the conventional UB concentration measuring method requires a spectrophotometer for measuring the absorbance and a cumbersome procedure and therefore can not be practiced immediately when desired at all desired places.

DISCLOSURE OF THE INVENTION

The apparatus of this invention for measuring the concentration of bilirubin comprises a measuring thermostat assembly for maintaining a measuring cell at a specified temperature as accommodated therein, a light source assembly for projecting light into the measuring cell in the thermostat assembly, a light receiving assembly for converting to an electric signal the intensity of monochromatic light having a predetermined wavelength and passing through the measuring cell, a unit for measuring the amount of bilirubin by continuously converting the output of the light receiving assembly to

absorbance for the monochromatic light, a TB concentration memory circuit for storing the output of the bilirubin amount measuring unit produced when a serum is placed in the cell as the concentration of total bilirubin in the serum, a UB concentration measuring unit for calculating the concentration of bilirubin unbound to albumin in the serum by measuring the rate of variation in the output of the bilirubin amount measuring unit after both peroxide and hydrogen peroxide or a hydrogen peroxide producing substance have been added to the serum in the measuring cell, and displays for showing the output of the TB concentration memory circuit and the output of the UB concentration measuring unit. Accordingly the TB concentration and UB concentration of the serum can be measured simultaneously by a very simple procedure including inserting the measuring cell into the thermostat assembly, placing the serum and prepared reagent into the cell and depressing, for example, a push-button switch. Moreover, the displays immediately show the measurements. It is therefore possible to conduct measurements immediately at any place, for example, at the bedside. It is clinically of great significance in the field of pediatrics or obstetrics to simultaneously measure the TB concentration and UB concentration of the same serum. Further if an urgent examination is conducted by measuring these concentrations at the bedside, the examination permits early detection of kernicterus in newborn infants, thus playing an important role in checking immature infants for health.

The measuring thermostat assembly of the bilirubin concentration measuring apparatus of the invention is provided with a rotary magnet positioned below the measuring cell. Accordingly the rotation of the magnet rotates an iron piece or the like placed in the cell, automatically instantaneously agitating a buffer solution in the cell along with the serum and reagent added thereto.

Further with the measuring apparatus of the invention, the light receiving assembly converts to electric signals the intensities of two kinds of monochromatic light beams of different wavelengths, and the bilirubin amount measuring unit calculates the amount of bilirubin based on the absorbances for the two kinds of monochromatic light beams. Consequently the influence of hemoglobin liberated into the serum can be compensated for to give accurate measurements.

Further with the measuring apparatus of the invention, the bilirubin measuring unit has the function of adjusting to zero the absorbance for the monochromatic light before the serum is placed into the measuring cell. With this zero adjustment made for every measurement, the absorbance for the monochromatic light after the serum has

5

0071650

been placed in can be measured accurately to afford accurate concentration measurements.

Furthermore the concentration measuring apparatus of the invention is so adapted that when the output of the bilirubin amount measuring unit has reduced from the concentration of total bilirubin by a specified ratio, the UB concentration measuring unit automatically starts to measure the rate of variation in the output. This assures accuracy in measuring the UB concentration and high reproducibility by eliminating the differences due to different persons.

### BIREF DESCRIPTION OF THE DRAWINGS

Fig. 1 is a perspective view showing the appearance of an apparatus of the invention for measuring the concentration of bilirubin;

Fig. 2 is a diagram showing the construction of the apparatus; and

Fig. 3 shows measuring steps with use of the apparatus.

### BEST MODE OF CARRYING OUT THE INVENTION

The present invention will be described below in greater detail with reference to the accompanying drawings.

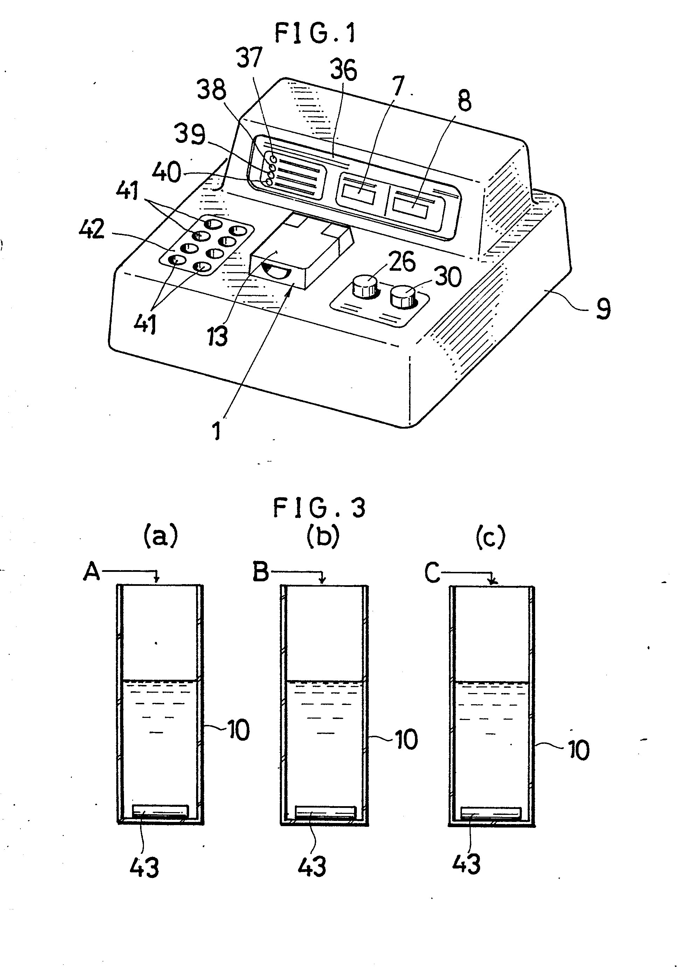

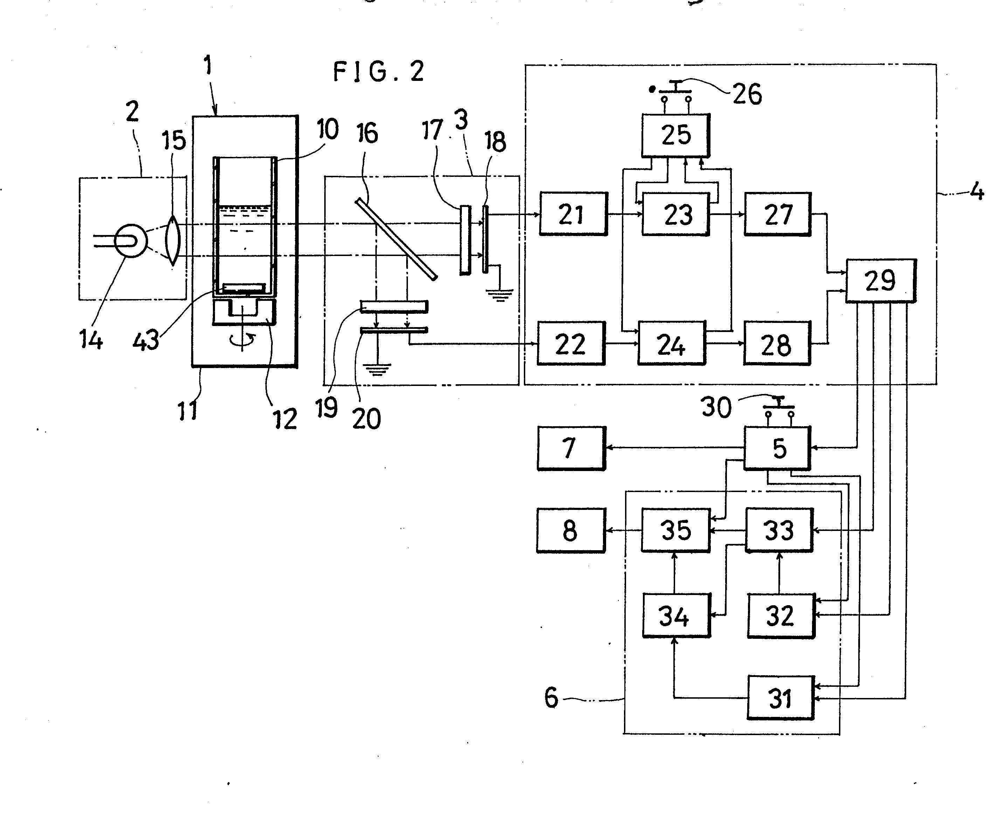

Fig. 1 and Fig. 2 respectively show the appearance and construction of a bilirubin concentration measuring apparatus. The apparatus comprises a measuring thermostat assembly 1, a light source assembly 2, a light receiving assembly 3, a bilirubin amount measuring unit 4, a TB concentration memory circuit 5, a UB concentration measuring unit 6, a TB concentration display 7 and a UB concentration display 8. These components are assembled as accommodated in a single case 9.

The measuring thermostat assembly 1 comprises a thermostat 11 for accommodating a measuring cell 10 and maintaining the cell at a predetermined temperature, and a rotary magnet 12 positioned below the measuring cell 10 placed in the thermostat 11. The thermostat 11 is disposed in the center of the interior of the case 9 and has a cover 13 on the upper surface of the case 9.

The light source assembly 2 comprises a tungsten lamp 14 disposed beside the measuring cell 10 placed in the thermostat assembly 1, and a lens 15 interposed between the cell 10 and the lamp 14, whereby parallel rays are made incident on the measuring cell 10.

The light receiving assembly 3 is disposed within the case 9 on one side of the thermostat assembly 1 opposite to the light source assembly 2. The light receiving assembly 3 comprises a half mirror 16 for dividedly leading the rays passing through the cell 10 into two optical paths, an interference filter 17 for separating off a first beam of monochromatic light having a specified wavelength (460 nm) from one of the divided bundles of rays, a photocell 18 for converting the intensity of the first beam of monochromatic light to an electric signal, an interference filter 19 for separating off a second beam of monochromatic light having a different specified wavelength (575 nm) from the other divided bundle of rays, and a photocell 20 for converting the intensity of the second beam of monochromatic light to an electric signal. Photodiodes or the like are used as the photocells 18, 20.

The bilirubin amount measuring unit 4 comprises two current-voltage converters 21, 22, two automatic zero adjustment circuits 23, 24, an automatic zero adjustment control circuit 25, a push-button switch 26 for automatic zero adjustment, two amplifiers 27, 28 and a differential circuit 29. Of these components, the push-button switch 26 only is disposed on the upper surface of the case 9, with the other components housed in the case 9.

The TB concentration memory circuit 5 is accommodated in the case 9 and connected to a TB concentration measuring push-button switch 30 provided on the upper surface of the case 9.

The UB concentration measuring unit 6 comprises a start control circuit 31 for UB concentration measurement, a UB concentration measurement termination value memory circuit 32, a comparison circuit 33, a timer circuit 34 and a UB concentration calculation circuit 35. These components are accommodated in the case 9.

The TB concentration display 7 and the UB concentration display 8 are arranged on a display panel 36 formed on the case 9 integrally therewith.

The display panel 36 is provided with a measurement preparation display lamp 37, an automatic zero adjustment display lamp 38, a TB concentration measurement display lamp 39 and a UB concentration measurement display lamp 40.

A preparation thermostat assembly 42 formed with a plurality of measuring cell accommodating cavities 41 is provided in the case 9. Each cavity 41 has an upper end opening in the upper surface of the case 9.

Fig. 3 shows measuring steps with use of the bilirubin concentration measuring apparatus described above. The procedure for measuring TB concentration and UB concentration will be described next with reference to Fig. 2 and Fig. 3.

When the power supply for the measuring apparatus is turned on first, the measurement preparation display lamp 37 goes on, and the preparation thermostat assembly 42 and the measuring thermostat assembly 1 are warmed up. Upon the thermostat assemblies 1, 42 reaching a predetermined temperature ($30^\circ$ C), the preparation display lamp 37 goes off. A 1 ml quantity of potassium phosphate buffer solution A is then placed into the measuring cell 10 (see Fig. 3 (a)), and the cell 10 is inserted into a cavity 41 of the preparation thermostat assembly 42. The buffer solution has 20 µg glucose previously added thereto. Further the measuring cell 10 has placed therein a stirring member 43 in the form of a small round bar of iron.

When the buffer solution has reached a specified temperature, the measuring cell 10 is withdrawn from the thermostat assembly 42 and inserted into the thermostat 11 of the measuring thermostat assembly 1, and the cover 13 is closed. The automatic zero adjustment push-button switch 26 is depressed to set the absorbances for the first and second beams of monochromatic light to zero. The light incident on the cell 10 from the light source assembly 2 passes through the buffer solution, with the result that a very small current in proportion to the intensity of the first monochromatic light and a very small current proportional to the intensity of the second monochromatic light flow from the two photocells 18, 20 of the light receiving assembly 3 into the two converters

0071650

21, 22 of the bilirubin amount measuring unit 4 respectively individually. The small currents from the photocells 18, 20 are logarithmically converted to voltages
by the converters 21, 22, whereby the transmittances of
the solution in the cell 10 for the monochromatic light
beams are converted to absorbances. The outputs from
the converters 21, 22 are fed to the two automatic zero
adjustment circuits 23, 24 respectively. The zero
adjustment circuits 23, 24 are in condition for receiving
output voltages from the control circuit 25. Signals
in proportion to the differences between the output
voltages of the converters 21, 22 and the output voltages
of the control circuit 25 are fed to the control circuit
25 and to the two amplifiers 27, 28 individually in
corresponding relation. With the push-button switch 26
depressed, the outputs of the control circuit 25 vary,
and when the output voltages become equal to the output
voltages of the converters 21, 22, the outputs from the
adjustment circuits 23, 24 become zero. The control
circuit 25 detects this and maintains its output voltages
for the circuits 23, 24 at the values concerned. The
outputs of the adjustment circuits 23, 24 are therefore
zero at this time. When the outputs of the converters
21, 22 thereafter vary, the differences between these
outputs and the constant voltages from the control circuit 25 are delivered from the adjustment circuits 23, 24 respectively. Incidentally when the push-button switch 26 is depressed, the automatic zero adjustment display lamp 38 goes on, while when the switch 26 is released from the hand, the lamp 38 goes off.

After the completion of automatic zero adjustment, 25 μℓ of serum B is placed into the measuring cell 10 (see Fig. 3 (b)). The bush-button switch 30 is depressed to measure the TB concentration. The serum added to the buffer solution is quickly agitated by the stirring member 43. The first monochromatic light is absorbed by the bilirubin in the serum, and a voltage in proportion to the absorbance, i.e. the amount of bilirubin, is delivered from the first automatic zero adjustment circuit 23. If the serum is free of hemolysis, the first monochromatic light is absorbed only by bilirubin, but if the serum involves hemolysis, the light is absorbed by bilirubin and also by hemoglobin. To eliminate the influence of absorption by hemoglobin, the amount of hemoglobin is measured with use of the second monochromatic light. Thus the second monochromatic light is absorbed by hemoglobin, and a voltage in proportion to the absorbance, i.e. the amount of hemoglobin, is delivered from the second automatic zero adjustment circuit 24. The outputs from the two circuits 23, 24 are amplified by the amplifiers 27, 28. The outputs from the amplifiers 27, 28 are fed to the differential circuit 29 for subtraction, whereby the influence of hemoglobin is compensated for. Thus the differential circuit 29 gives the amount of bilirubin. When the push-button switch 30 is depressed, the amount of bilirubin given by the differential circuit 29 at that time is stored in the memory circuit 5 as the TB concentration. This value is shown on the TB concentration display 7, and the TB concentration measurement display lamp 39 goes on. Once the TB concentration has been stored, the output of the memory circuit 5 and the value shown on the display 7 remain unchanged even if the output of the differential circuit 29 thereafter alters. Further simultaneously with the depression of the push button switch 30, a UB concentration measurement start value corresponding to 95% of the TB concentration is stored in the start control circuit 31, and a UB concentration measurement termination value corresponding to 80% of the TB concentration is stored in the memory circuit 32.

After the TB concentration has been measured, 20 $\mu\ell$ of a reagent C prepared from glucose oxidase and peroxidase is placed into the measuring cell 10 to measure the UB concentration. When the reagent is added to the serum in the cell 10, the peroxidase starts to

13    0071650

oxidize and decompose UB in the presence of hydrogen peroxide produced by glucose and glucose oxidase, gradually reducing the amount of serum bilirubin, i.e. the output of the differential circuit 29. The start control circuit 31, monitoring the output of the differential circuit 29 at all times, gives the timer circuit 34 a time measurement start signal when this output has reached the previously stored start value. Consequently the timer circuit 34 starts measuring time for measuring the rate of variation in the amount of bilirubin. On the other hand, the comparison circuit 33, comparing the output of the differential circuit 29 with the termination value stored in the memory circuit 32 at all times, delivers a time measurement termination signal to the timer circuit 34 when the amount of bilirubin has reduced to the termination value. At the same time, the circuit 29 gives a calculation signal to the UB concentration calculation circuit 35. At this time, the timer circuit 34 has measured the time taken for the bilirubin amount, as reduced to the start value, to further decrease to the termination value. The rate of variation in the bilirubin amount is therefore determined from the measurement. The calculation circuit 35 calculates the UB concentration based on the result. When peroxidase is caused to act on serum bilirubin in the presence of hydrogen peroxide, the rate of reduction in the amount of bilirubin is in
a definite proportional relation to the UB concentration
as already stated, so that the UB concentration can be
calculated if the rate of variation in the bilirubin amount
is determined.  The UB concentration thus calculated is
shown and retained on the UB concentration display 8 and,
at the same time, the measurement display lamp 40 goes
on to complete the measurement.

The components of the measuring apparatus are
not limited to those of the foregoing embodiment in
construction but can be modified suitably.

While the TB concentration and the UB concentration are separately shown on the two displays 7, 8
arranged side by side in the above embodiment, these two
concentrations may be alternatively shown on a single
display.

With the above embodiment, the bilirubin amount
measuring unit 4 has an automatic zero adjustment function
afforded by the automatic zero adjustment circuits 23, 24,
automatic zero adjustment control circuit 25 and push-
button switch 26 and is therefore adapted for automatic
zero adjustment for every measurement, so that the
absorbance for monochromatic light can be measured accurately to assure very high accuracy for measurements.
However, the zero adjustment means may be of the manual type or may be provided in the rear of the differential circuit 29 or some other suitable location. If exceedingly high measurement accuracy is not required, the zero adjustment means can be dispensed with.

According to the foregoing embodiment, the amount of serum bilirubin is calculated based on the absorbances for two kinds of monochromatic light beams to compensate for the influence of liberated hemoglobin and thereby assure greatly improved measurement accuracy. However, if very high accuracy is not required, the first monochromatic light beam only is usable for the determination of bilirubin.

The start value and the termination value for measuring the UB concentration vary with the amount of serum, the concentration of reagent, etc. and are not limited to 95% and 80%, respectively, of the TB concentration adopted for the foregoing embodiment. Although the rate of variation in the bilirubin amount is determined by measuring the time taken for the bilirubin amount to decrease by a predetermined quantity, the variation rate can be determined also by measuring the ratio of reduction of the bilirubin amount upon the lapse of a specified period of time. Further with the above embodiment, the measurement of the variation rate of the bilirubin amount is automatically started when the

0071650

amount has reached a UB concentration measurement start value after decreasing from the TB concentration by a predetermined ratio and therefore achieves high accuracy free of person-to-person differences, but unless very high measurement accuracy is required, the measurement of the variation rate can be started, for example, by depressing a push-button switch simultaneously with the placement of the reagent into the measuring cell 10 after measuring the TB concentration.

With the foregoing embodiment, glucose is added to the buffer solution in the measuring cell 10 first, and the reagent prepared from glucose oxidase and peroxidase is thereafter added thereto when the UB concentration is to be measured, but both glucose and reagent may be added when the UB concentration is to be measured. Although glucose and glucose oxidase are used for producing hydrogen peroxide according to the embodiment described, hydrogen peroxide may be added directly, or an enzyme or some other substance capable of producing hydrogen peroxide may be used. The temperature of the measuring thermostat assembly 1 is not limited to that mentioned for the embodiment.

INDUSTRIAL APPLICABILITY

The bilirubin concentration measuring apparatus of this invention is suited for use in clinical tests for measuring the TB concentration and UB concentration of the serum.

CLAIMS

1. A bilirubin concentration measuring apparatus characterized in that the apparatus comprises a measuring thermostat assembly 1 for maintaining a measuring cell 10 at a specified temperature as accommodated therein, a light source assembly 2 for projecting light into the measuring cell 10 in the thermostat assembly 1, a light receiving assembly 3 for converting to an electric signal the intensity of monochromatic light having a predetermined wavelength and passing through the measuring cell 10, a unit 4 for measuring the amount of bilirubin by continuously converting the output of the light receiving assembly 3 to absorbance for the monochromatic light, a TB concentration memory circuit 5 for storing the output of the bilirubin amount measuring unit 4 produced when a serum is placed in the cell 10 as the concentration of total bilirubin in the serum, a UB concentration measuring unit 6 for calculating the concentration of bilirubin unbound to albumin in the serum by measuring the rate of variation in the output of the bilirubin amount measuring unit 4 after both peroxide and hydrogen peroxide or a hydrogen peroxide producing substance have been added to the serum in the measuring cell 10, and displays 7, 8 for showing the output of the TB concentration memory circuit 5 and the output of the UB concentration measuring unit 6.

2. A bilirubin concentration measuring apparatus as defined in claim 1 wherein the measuring thermostat assembly 1 is provided with a rotary magnet 12 positioned below the measuring cell 10.

3. A bilirubin concentration measuring apparatus as defined in claim 1 wherein the light receiving assembly 3 converts to electric signals the intensities of two kinds of monochromatic light beams having different wavelengths, and the bilirubin amount measuring unit 4 calculates the amount of bilirubin based on the absorbances for the two kinds of monochromatic light beams.

4. A bilirubin concentration measuring apparatus as defined in claim 3 wherein the light receiving assembly 3 comprises a half mirror 16 for dividedly leading the rays passing through the cell 10 into two optical paths, an interference filter 17 for separating off a first beam of monochromatic light having a specified wavelength from one of the divided bundles of rays, a photocell 18 for converting the intensity of the first beam of monochromatic light to an electric signal, an interference filter 19 for separating off a second beam of monochromatic light having a different specified wavelength from the other divided bundle of rays, and a photocell 20 for converting the intensity of the second beam of monochromatic light to an electric signal.

5. A bilirubin concentration measuring apparatus as defined in claim 4 wherein the first beam of monochromatic light has a wavelength of 460 nm, and the second beam of monochromatic light has a wavelength of 575 nm.

6. A bilirubin concentration measuring apparatus as defined in claim 1 wherein the bilirubin amount measuring unit 4 has a zero adjustment function for reducing the absorbance for the monochromatic light to zero before the serum is placed into the measuring cell 10.

7. A bilirubin concentration measuring apparatus as defined in claim 1 wherein the UB concentration measuring unit 6 automatically starts to measure the rate of variation in the output of the bilirubin amount measuring unit 4 when the output has reduced from the concentration of total bilirubin by a specified ratio.

FIG.1

FIG.3

(a)     (b)     (c)

FIG. 2

0071650

# INTERNATIONAL SEARCH REPORT

0071650

International Application No  PCT/JP82/00041

**I. CLASSIFICATION OF SUBJECT MATTER** (if several classification symbols apply, indicate all) [3]

According to International Patent Classification (IPC) or to both National Classification and IPC

Int. Cl.[3]   G01N 21/75, G01N 33/72, C12Q 1/28

**II. FIELDS SEARCHED**

| Minimum Documentation Searched [4] | |
|---|---|
| Classification System | Classification Symbols |
| I P C | G01N 21/75, G01N 21/77, G01N 21/78, C12Q 1/28, G01N 21/25, G01N 33/72 |

| Documentation Searched other than Minimum Documentation to the Extent that such Documents are Included in the Fields Searched [5] |
|---|
| Jitsuyo Shinan Koho          1926 - 1981 |
| Kokai Jitsuyo Shinan Koho    1971 - 1981 |

**III. DOCUMENTS CONSIDERED TO BE RELEVANT** [14]

| Category [*] | Citation of Document, [16] with indication, where appropriate, of the relevant passages [17] | Relevant to Claim No. [18] |
|---|---|---|
| A | JP,A, 48-101195 (Nemoto Sakayuki) 20. December. 1973   (20.12.73) | 1 - 7 |
| A | JP,A, 50-47693 (F. Hoffmann-La Roche & Co. A.G.) 28. April. 1975   (28.4.75) | 1 - 7 |

[*] Special categories of cited documents: [15]

"A" document defining the general state of the art

"E" earlier document but published on or after the international filing date

"L" document cited for special reason other than those referred to in tne other categories

"O" document referring to an oral disclosure, use, exhibition or other means

"P" document published prior to the international filing date but on or after the priority date claimed

"T" later document published on or after the international filing date or priority date and not in conflict with the application, but cited to understand the principle or theory underlying the invention

"X" document of particular relevance

**IV. CERTIFICATION**

| Date of the Actual Completion of the International Search [3] | Date of Mailing of this International Search Report [3] |
|---|---|
| May 10, 1982   (10.05.82) | May 17, 1982   (17.05.82) |
| International Searching Authority [1] | Signature of Authorized Officer [10] |
| Japanese Patent Office | |

Form PCT/ISA/210 (second sheet) (October 1977)